# FASCICULE DE BREVET EUROPEEN

(11) **EP 3 723 851 B1**
(45) Date de publication et mention de la délivrance du brevet: **01.03.2023**
(21) Numéro de dépôt: 18833908.9
(22) Date de dépôt: 03.12.2018
(51) Int. Cl.: A61N 5/06, A61B 18/22

(54) **DISPOSITIF D'ILLUMINATION LOCALISÉE IMPLANTABLE À ARCHITECTURE AMÉLIORÉE**
IMPLANTIERBARE, LOKALISIERTE BELEUCHTUNGSVORRICHTUNG MIT VERBESSERTER ARCHITEKTUR
IMPLANTABLE LOCALISED ILLUMINATING DEVICE WITH IMPROVED ARCHITECTURE

(30) Priorité: 11.12.2017 FR 1761885; 11.12.2017 FR 1761886; 11.12.2017 FR 1761888
(43) Date de publication de la demande: 21.10.2020
(73) Titulaire: Commissariat à l'Énergie Atomique et aux Énergies Alternatives, 75015 Paris (FR)
(72) Inventeur: CHABROL, Claude, 38320 Poisat (FR); RENAULT, Sarah, 38700 Corenc (FR)
(74) Mandataire: INNOV-GROUP
(86) Numéro de dépôt international: PCT/FR2018/053084
(87) Numéro de publication internationale: WO 2019/115909

(56) Documents cités:
- WO-A1-86/03293
- WO-A1-2016/102351
- WO-A1-2017/025423
- WO-A2-2007/120678
- US-A1- 2014 074 182
- US-B2- 7 003 184

## Description

### Domaine technique de l'invention

La présente invention se rapporte à un dispositif d'illumination destiné à être implanté au moins en partie dans un être vivant pour illuminer de manière localisée au moins une zone de l'être vivant.

### Etat de la technique

Pour le traitement de certaines pathologies d'un être vivant, il a été imaginé d'aller stimuler de manière optique une zone interne d'un être vivant. Pour cela, il a été proposé des dispositifs qui comportent une source de lumière et qui sont implantés au moins en partie ou totalement dans l'être vivant pour aller illuminer la zone souhaitée.

On s'est notamment aperçus de l'intérêt de tels dispositifs pour illuminer/irradier de manière optique certaines zones du cerveau humain.

Cependant, compte-tenu des risques liés à l'implantation d'un tel dispositif dans le cerveau, on comprend qu'un tel dispositif doive être parfaitement conçu.

Un tel dispositif est connu de la demande de brevet US2012/259393A1. La solution présentée dans ce document n'est pas aboutie.

Une autre solution est connue de la demande de brevet WO2016/102351A1. La solution présentée dans ce document s'avère peu flexible et difficile à mettre en oeuvre sans risquer des lésions au niveau des tissus du patient implanté.

D'autres solutions sont également décrites dans les documents US2014/074182A1 et US7003184B2.

Le but de l'invention est de proposer un dispositif d'illumination destiné à être implanté au moins en partie dans un être vivant, notamment pour illuminer une ou plusieurs zones de son cerveau, ledit dispositif présentant une architecture adaptée pour remplir un tel rôle. Il devra notamment remplir un ou plusieurs des objectifs suivants :
- Employer des matériaux bio-compatibles ;
- Minimiser les risques médicaux lors de son implantation et explantation ;
- Assurer le confort esthétique et physique du patient ;
- Etre facile à fabriquer et d'une fabrication facilement reproductible ;
- Permettre un traitement efficace ;

### Exposé de l'invention

Ce but est atteint par un dispositif d'illumination implantable, destiné à être implanté dans un être vivant pour illuminer de manière localisée une zone dudit être vivant, ledit dispositif comportant une sonde présentant une architecture allongée suivant un axe entre une extrémité proximale et une extrémité distale, ladite sonde se présentant sous la forme d'une tige de section circulaire de diamètre constant sur toute sa longueur, ladite architecture étant réalisée à partir de :
- une fibre optique de stimulation qui comporte un coeur, un revêtement et une gaine de protection,
- un enrobage,
- ladite architecture comportant :
- plusieurs tronçons successifs suivant son axe longitudinal, chaque tronçon présentant une section à plusieurs couches superposées de manière concentrique, dans lesquelles chaque tronçon comporte une section à plusieurs couches superposées distincte de la section de chaque tronçon adjacent,
- au moins un premier tronçon le long duquel ladite fibre optique de stimulation est enrobée par l'enrobage,
- au moins un deuxième tronçon prolongeant ledit premier tronçon, dans lequel ladite fibre optique de stimulation est dénudée pour retirer sa gaine de protection de manière à laisser uniquement son coeur et son revêtement optique enrobé par l'enrobage,
- au moins un tronçon terminal dont la section comporte uniquement ledit enrobage, ledit tronçon terminal comportant l'extrémité distale de la sonde et en ce que cette extrémité distale est configurée de manière à être atraumatique.

Selon une première réalisation particulière, qui est revendiquée dans les revendications attenantes, la section du premier tronçon comporte une première couche comprenant le coeur de fibre optique, une deuxième couche comprenant le revêtement de fibre optique, une troisième couche comprenant la gaine de protection de fibre optique, une quatrième couche comprenant l'enrobage et une cinquième couche externe composée d'un tube. La section du deuxième tronçon prolongeant ledit premier tronçon comporte une première couche comprenant le coeur de ladite fibre optique, une deuxième couche formée du revêtement de la fibre optique, une troisième couche comprenant ledit enrobage, le dispositif comporte également une quatrième couche comprenant ledit tube. Le dispositif comporte un troisième tronçon prolongeant ledit deuxième tronçon et dont la section comporte une première couche comprenant ledit enrobage et une deuxième couche comprenant ledit tube.

Selon une particularité de l'invention le tube est réalisé dans un matériau radio-opaque.

Selon une deuxième réalisation particulière, qui n'est pas revendiquée et qui est montrée uniquement à titre illustratif, la section du premier tronçon comporte une première couche comprenant le coeur de fibre optique, une deuxième couche comprenant le revêtement de fibre optique, une troisième couche comprenant la gaine de protection de fibre optique et une quatrième couche comprenant l'enrobage qui est surmoulée sur la fibre optique.

Selon une particularité de cette deuxième réalisation non revendiquée, le deuxième tronçon prolonge ledit premier tronçon et présente une section qui comporte uniquement une première couche comprenant le coeur de ladite fibre optique, une deuxième couche formée du revêtement de la fibre optique, une troisième couche comprenant ledit enrobage.

Selon une particularité du dispositif, il comporte un tronçon de connexion, situé en amont du premier tronçon, dont la section comporte une première couche formée du coeur de la fibre, une deuxième couche formée du revêtement de fibre optique, une troisième couche formée d'une colle et une quatrième couche formée d'une férule.

Selon une autre particularité, le dispositif comporte une source de lumière sur laquelle est connectée de manière optique ladite sonde par son extrémité proximale.

Selon une autre particularité, le dispositif comporte un organe de butée associé à au moins une entretoise pour régler l'enfoncement de la sonde et la position de son extrémité distale.

Selon une autre particularité, le dispositif comporte au moins un organe de centrage de ladite fibre optique de stimulation dénudée, positionné autour de ladite fibre optique et agencé pour former une cale pour ladite fibre optique dénudée.

Selon une autre particularité, ledit organe de centrage comporte un manchon cylindrique présentant un canal central dans lequel est insérée ladite fibre optique dénudée.

Selon une autre particularité, ledit manchon comporte une ou plusieurs rainures latérales externes.

Selon une variante de réalisation, ledit organe de centrage comporte une pièce en forme d'hélice venant s'enrouler autour du coeur de la fibre optique et de son revêtement.

Selon une particularité, ledit organe de centrage est réalisé dans un matériau de type radio-opaque.

Selon une particularité, le dispositif comporte un système de retour optique comprenant au moins une première fibre optique de retour comprenant une extrémité optique d'entrée configurée pour capter un signal optique et une extrémité optique de sortie configurée pour transmettre le signal optique capté, ladite première fibre optique de retour étant enroulée en hélice autour de la fibre optique de stimulation sur au moins une partie de la longueur de ladite fibre optique de stimulation.

Selon une autre particularité, le système de retour optique comporte une deuxième fibre optique de retour enroulée en hélice autour de ladite fibre optique de stimulation et comportant une extrémité optique d'entrée et une extrémité optique de sortie.

Selon une autre particularité, l'extrémité optique d'entrée de la première fibre optique de retour et l'extrémité optique d'entrée de la deuxième fibre optique de retour sont situées à des longueurs distinctes de la sortie optique de la fibre optique de stimulation.

Selon une autre particularité, chaque fibre optique de retour comporte un coeur de section circulaire et un revêtement optique présentant une section sensiblement carrée, présentant deux côtés opposés recourbés, l'un de ces deux côtés présentant un premier rayon de courbure et l'autre de ces deux côtés opposés présentant un deuxième rayon de courbure distinct du premier rayon de courbure.

L'invention concerne également un procédé de fabrication d'un dispositif d'illumination implantable tel que défini ci-dessus dans sa première réalisation particulière, ledit procédé comportant les étapes suivantes :
- Remplissage du tube par le matériau d'enrobage non réticulé,
- Séchage au moins partiel du matériau d'enrobage non réticulé pour le figer à l'intérieur du tube,
- Dépôt d'une quantité calibrée de matériau d'enrobage non réticulé pour former ledit tronçon terminal,
- Réticulation du matériau d'enrobage sur toute la longueur de la sonde.

### Brève description des figures

D'autres caractéristiques et avantages vont apparaître dans la description détaillée qui suit, faite en regard des dessins annexés listés ci-dessous :
- La figure 1A représente, vue suivant une coupe longitudinale axiale et plusieurs coupes transversales, l'architecture du dispositif d'illumination implantable de l'invention, selon une première réalisation ;
- La figure 1B représente, vue suivant une coupe longitudinale axiale et plusieurs coupes transversales, l'architecture du dispositif d'illumination implantable selon une deuxième réalisation non revendiquée ;
- La figure 2 représente la structure multicouches d'une fibre optique de type HCS ;
- Les figures 3A à 3C représentent différentes variantes de réalisation d'un organe de centrage employé dans la sonde du dispositif de l'invention
- Les figures 4A à 4C représentent différentes variantes de réalisation d'un système de retour optique employé dans le dispositif de l'invention ;
- La figure 5 illustre différentes solutions de réalisation de la sortie optique ;
- Les figures 6A et 6B illustrent un principe de réglage du positionnement de la sonde ;

### Description détaillée d'au moins un mode de réalisation

L'invention concerne un dispositif d'illumination implantable.

Il aura pour objectif de réaliser une illumination des tissus dans un but de neuroprotection, de stimulation, ou dans un but opto-génétique.

Le dispositif sera notamment parfaitement adapté pour assurer une illumination ventriculaire du cerveau de manière localisée. Il pourra servir dans le traitement des maladies neurodégénératives telles que la maladie de Parkinson. Bien entendu, il pourra s'appliquer à d'autres pathologies et pourra être employé pour une implantation dans toute zone du corps d'un être vivant.

Le dispositif est avantageusement décrit pour la stimulation optique d'une zone du cerveau.

La particularité du dispositif de l'invention est notamment de permettre une stimulation optique à partir d'une solution souple, suffisamment fine (1 à 2,5mm, idéalement compris entre 1,25 et 1,3 pour être adapté aux outillages de DBS-"Deep Brain Stimulation") pour être insérée dans le troisième ventricule ou en contact de toute autre zone du cerveau accessible chirurgicalement sans outil stéréotaxique, une fois qu'un guide ou cathéter ventriculaire (neuro-guide Renishaw ou cathéter ventriculaire équipé d'un Rickham-Ommaya, ou toute autre solution de guidage adaptée) a été préalablement positionné entre la boite crânienne et le troisième ventricule.

Le dispositif comporte une sonde 1. La sonde 1 présente une architecture allongée suivant un axe longitudinal lorsqu'elle n'est pas coudée. Autrement dit, la sonde se présente sous la forme d'une tige présentant un certain degré de flexibilité et de déformation élastique. La tige comporte ainsi deux extrémités, une extrémité proximale formant une entrée optique destinée à recevoir un rayonnement lumineux en provenance d'une source de lumière 2 et une extrémité distale comprenant une sortie optique par laquelle le rayonnement lumineux est transmis en direction de la zone à illuminer/traiter.

La sonde pourra se déformer en flexion de manière élastique pour former un angle compris entre 30° et 110° par rapport à sa forme initiale droite à l'état repos, préférentiellement de 90° formant alors un coude au niveau du passage entre l'extérieur du crâne et le cerveau.

La source de lumière 2 fait avantageusement partie du dispositif.

La source pourra émettre dans une gamme de longueur d'onde comprise entre 650nm et 1070nm dans le cadre de la neuroprotection ou 450nm à 650 nm pour l'optogénétique. La source 2 sera avantageusement alimentée par une batterie et commandée par une unité de commande 3 de manière à générer un courant électrique continu ou des impulsions à une fréquence donnée. La source pourra être intégrée à un neuro-stimulateur ou être déportée.

La source de lumière 2 peut être une diode laser, une diode électroluminescente ou toute autre solution adaptée à un couplage optique dans la sonde.

L'unité de commande 3 de ladite source de lumière 2 fait avantageusement partie du dispositif.

La source de lumière 2 et l'unité de commande 3 peuvent être réunies dans un même boîtier.

Le dispositif peut comporter une embase optique reliée à la source de lumière. L'embase optique peut être agencée sur ledit boîtier.

La sonde 1 comporte un connecteur complémentaire formant son entrée optique, agencé pour se connecter (respectivement déconnecter) sur ladite embase optique.

La tige formant la sonde 1 présente avantageusement une section circulaire de diamètre externe qui pourra être compris entre 1 et 2,5 mm selon le mode de réalisation.

Le dispositif présente une architecture parfaitement étanche. Le diamètre de la sonde est avantageusement constant sur toute la longueur de la sonde (hormis au niveau de son système de butée - voir ci-après).

L'extrémité de la sonde formant la sortie optique présente avantageusement une forme dite atraumatique. Il s'agira de lui donner une forme sans arête saillante. Il pourra par exemple s'agir d'une forme de type hémisphérique, oblongue ou équivalente.

Le long de son axe, l'architecture de la sonde 1 présente une section dont la composition à plusieurs couches concentriques varie.

La sonde comporte ainsi plusieurs tronçons (Sc à S4 ou Sc à S30) successifs entre son entrée optique et sa sortie optique. Chaque tronçon présente une structure spécifique à plusieurs couches concentriques.

La sonde comporte une fibre optique.

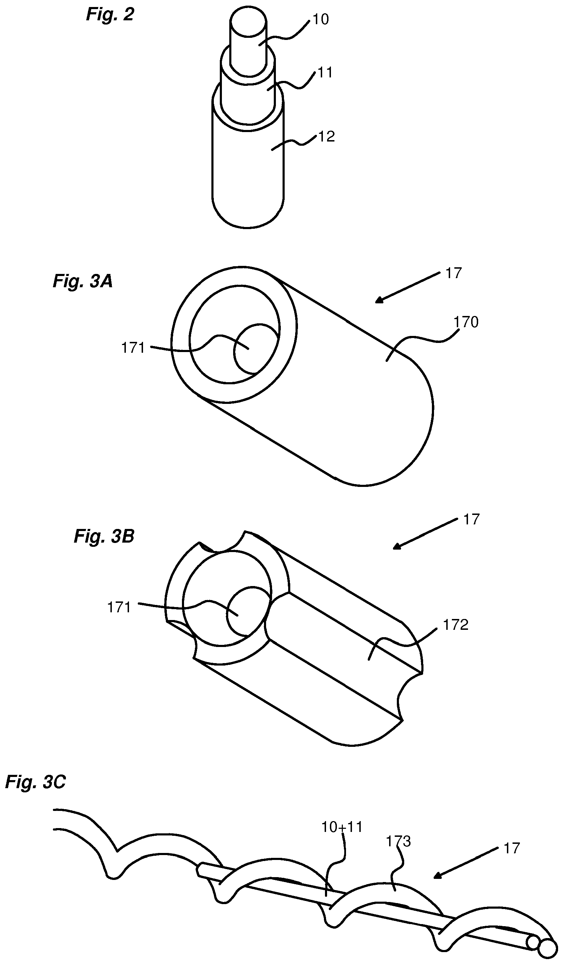

En référence à la figure 2, la fibre optique peut comporter un coeur 10 de type silice, de type saphir ou de type plastique (acrylate ou tout autre matériau adapté) et un revêtement 11 ("cladding") optique réalisé autour du coeur.

Le revêtement peut être réalisé en matériau plastique, par exemple dans un matériau de type fluoro-acrylate, dans un matériau de type silice ou autre matériau d'indice adapté.

La fibre optique peut comporter une gaine de protection 12 mécanique autour de son revêtement.

La gaine de protection 12 peut être réalisée dans un matériau de type éthylène tétrafluoroéthylène (ETFE).

Il s'agira ainsi de disposer par exemple d'une fibre optique de type HCS (Hard Clad Silica). Bien entendu, toute autre architecture pourrait être envisagée.

Ce type de fibre présente cependant un risque de décollement entre sa gaine en ETFE et son revêtement en Fluoro-acrylate.

Pour remédier à ce risque et pour améliorer la souplesse de la sonde, on propose de dénuder sur une longueur suffisante (par exemple 5 à 70mm) la fibre du côté distale. La fibre optique est ainsi dénudée pour retirer sa gaine de protection 12 et ne conserver que son coeur et son revêtement optique.

La longueur de dénudage est adaptée en fonction de la longueur implantée dans le cerveau, par exemple 70mm pour atteindre le plancher du 3ème ventricule.

De manière avantageuse, on propose également de dénuder la partie de la fibre optique collée dans la férule au niveau de l'entrée optique afin de garantir un bon centrage de la fibre dans ce connecteur.

Dans la suite de la description, la silicone employée pour réaliser le dispositif pourra être remplacé par un matériau souple de type polyuréthane présentant les mêmes qualités, notamment optiques et de stabilité dans le temps. Pour simplifier, nous ferons cependant référence à un matériau de type silicone.

A partir de la fibre telle que décrite ci-dessus, deux réalisations distinctes sont envisageables.

Dans une première réalisation selon l'invention, la sonde comporte un tube 14, par exemple réalisé en silicone, définissant un canal central. Le diamètre externe de la section du tube 14 définit le diamètre maximal de la section de la sonde (hormis au niveau de son système de butée - voir ci-après). La silicone formant le tube est choisie avec des caractéristiques mécaniques adaptées. Il pourra être de type optique ou non optique (c'est-à-dire transparent ou opaque). A titre d'exemple, le tube sera réalisé dans une silicone de type MED 4765.

La fibre optique vient s'insérer dans le canal central du tube 14. Sur toute la longueur de la sonde, de la silicone 13 est avantageusement ajoutée dans le tube 14, par injection ou autre principe de fabrication, de manière à venir enrober les différents éléments présents dans le tube et ainsi conférer une parfaite étanchéité à la sonde vis-à-vis de la zone traitée. Cette silicone injectée est avantageusement de type optique (c'est-à-dire disposant d'une transparence optique et d'un indice de réfraction adapté au coeur de la fibre optique et au liquide physiologique - indice de réfraction compris entre 1,33 et 1,46, préférentiellement 1,41 - silicone MED 6233).

La fibre peut être préalablement traitée (primer, traitement plasma...) pour garantir une bonne adhérence de la silicone 13, 130 lors de la fabrication.

Dans une deuxième réalisation non revendiquée, un surmoulage en silicone est réalisé directement autour de la fibre, venant l'enrober. La silicone surmoulée autour de la fibre vient ainsi conférer une parfaite étanchéité à la sonde vis-à-vis de la zone traitée.

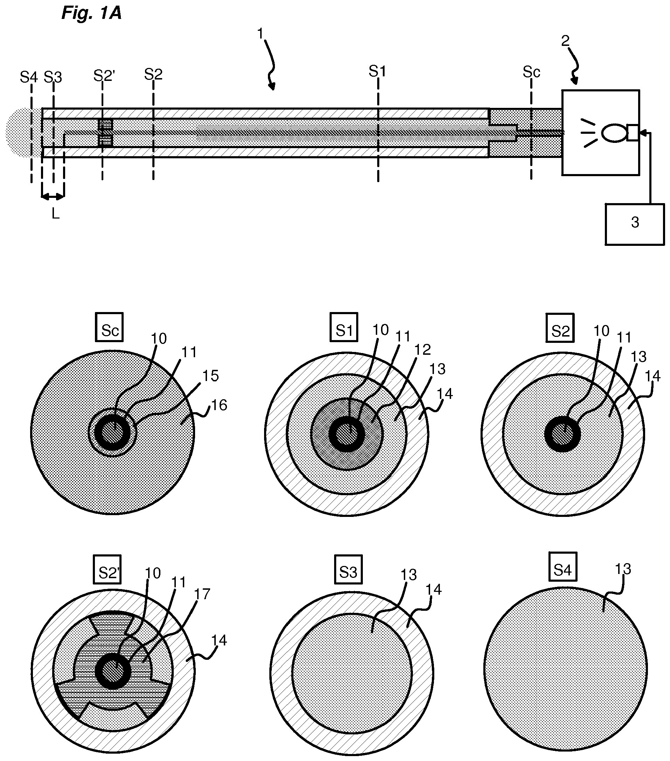

### Première réalisation - Figure 1A

De manière non limitative, en référence à la figure 1A, dans la première réalisation évoquée ci-dessus, la sonde présente ainsi différents tronçons, chaque tronçon présentant une section transversale spécifique. Cette architecture permet ainsi de donner différents niveaux de flexibilité, de protection mécanique et d'étanchéité à la sonde le long de son axe.

En partant de la couche interne la plus proche de l'axe et en allant vers la couche externe, la plus éloignée de l'axe de la sonde, les différents tronçons sont décrits ci-dessous.

La sonde comporte un tronçon de connexion (section Sc) formant avantageusement l'entrée optique du dispositif.

Le tronçon de connexion comporte :
- une première couche formée du coeur 10 de la fibre optique ;
- une deuxième couche formée du revêtement 11 de la fibre optique ;
- une troisième couche formée d'une colle 15 de type epoxy ;
- une quatrième couche formée d'une férule 16 permettant la connexion optique avec la source de lumière ; la colle epoxy permet de coller la férule sur la deuxième couche (revêtement de la fibre optique). Sur ce tronçon de connexion, on propose de dénuder la fibre optique sur au moins une partie de sa longueur pour retirer sa gaine de protection et permettre le collage de la férule.

La sonde comporte un premier tronçon (section S1), prolongeant le tronçon de connexion.

Ce premier tronçon comporte uniquement :
- une première couche formée du coeur 10 de la fibre ;
- une deuxième couche formée du revêtement 11 de la fibre optique ;
- une troisième couche formée de la gaine de protection 12 de la fibre optique ;
- une quatrième couche composée de l'enrobage 13 en silicone ;
- une cinquième couche formée du tube 14 ;

Ce premier tronçon est destiné à rester dans la zone extra-corticale de l'être vivant et sera coudé lors de son implantation à ce niveau. La première couche, la deuxième couche et la troisième couche forment la fibre optique telle que décrite ci-dessus. Pour améliorer la protection mécanique, la gaine de protection de la fibre est maintenue sur ce premier tronçon.

La sonde comporte un deuxième tronçon (section S2) prolongeant le premier tronçon dans le sens allant de l'entrée optique vers la sortie optique de la sonde.

Ce deuxième tronçon comporte uniquement :
- une première couche formée du coeur 10 de la fibre ;
- une deuxième couche formée du revêtement 11 de la fibre optique ;
- une troisième couche formée de l'enrobage 13 en silicone ;
- une quatrième couche formée du tube 14 ;

Le deuxième tronçon est destiné à pénétrer dans la zone intracérébrale. C'est sur ce deuxième tronçon que la fibre optique est dénudée pour retirer sa gaine de protection 12, permettant ainsi de rendre ce tronçon plus souple et étanche.

La sonde comporte un troisième tronçon (section S3) prolongeant le deuxième tronçon dans le sens allant de l'entrée optique vers la sortie optique de la sonde.

Ce troisième tronçon comporte uniquement :
- une première couche formée de l'enrobage 13 en silicone ;
- une deuxième couche formée du tube 14 ;

On remarque ainsi que l'extrémité de la fibre optique délimite la fin du deuxième tronçon et que le tube 14 se prolonge dans le troisième tronçon S3. La longueur L (typiquement 1.5mm à 2.5mm) entre l'extrémité du tube 13 et l'extrémité du coeur 10 de la fibre du côté distal, permet notamment de régler la densité de puissance en sortie de la sonde.

La sonde comporte un quatrième tronçon (section S4) prolongeant le troisième tronçon dans le sens allant de l'entrée optique vers la sortie optique de la sonde.

Ce quatrième tronçon comporte uniquement :
- une couche formée uniquement de l'enrobage 13 de silicone ;

Ce quatrième tronçon comporte l'extrémité de la sonde formant la sortie optique. On remarque que l'extrémité du tube délimite la fin du troisième tronçon.

Ce quatrième tronçon S4 se termine avantageusement par une extrémité atraumatique. La lumière issue du coeur de la fibre optique traverse ce quatrième tronçon. Selon l'application visée, ce quatrième tronçon peut comporter ou faire office de diffuseur optique.

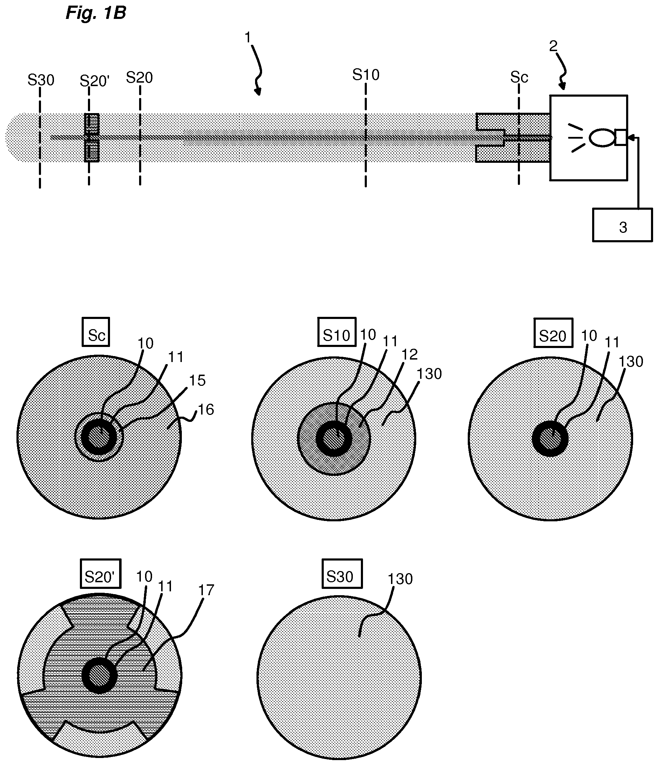

### Deuxième réalisation non revendiquée montrée à titre illustratif - Figure 1B

Cette deuxième réalisation diffère de la première réalisation en ce que le tube 14 est remplacé par un surmoulage complet en silicone. Le procédé employé sera ainsi distinct. Il ne s'agira pas de remplir le tube avec la silicone mais de réaliser un surmoulage en silicone autour de la fibre optique.

Dans cette deuxième réalisation, en partant de la couche interne la plus proche de l'axe et en allant vers la couche externe, la plus éloignée de l'axe de la sonde, les différents tronçons sont décrits ci-dessous.

La sonde comporte le même tronçon de connexion (section Sc) formant avantageusement l'entrée optique du dispositif.

Le tronçon de connexion comporte uniquement :
- une première couche formée du coeur 10 de la fibre optique ;
- une deuxième couche formée du revêtement 11 de la fibre optique ;
- une troisième couche formée d'une colle 15 de type epoxy ;
- une quatrième couche formée d'une férule 16 permettant la connexion optique avec la source de lumière ; la colle epoxy permet de coller la férule sur la deuxième couche (revêtement de la fibre optique) ;

Sur ce tronçon de connexion, on propose de dénuder la fibre optique sur au moins une partie de sa longueur pour retirer sa gaine de protection et permettre le collage de la férule.

La sonde comporte un premier tronçon (section S10), prolongeant le tronçon de connexion.

Ce premier tronçon comporte uniquement :
- une première couche formée du coeur 10 de la fibre ;
- une deuxième couche formée du revêtement 11 de la fibre optique ;
- une troisième couche formée de la gaine de protection 12 de la fibre optique ;
- une quatrième couche composée de l'enrobage 130 en silicone surmoulé autour de la fibre ;

Ce premier tronçon est destiné à rester dans la zone extra-corticale de l'être vivant et sera coudé lors de son implantation à ce niveau. La première couche, la deuxième couche et la troisième couche forment la fibre optique telle que décrite ci-dessus. Pour améliorer la protection mécanique, la gaine de protection de la fibre est maintenue sur ce premier tronçon.

La sonde comporte un deuxième tronçon (section S20) prolongeant le premier tronçon dans le sens allant de l'entrée optique vers la sortie optique de la sonde.

Ce deuxième tronçon comporte uniquement :
- une première couche formée du coeur 10 de la fibre ;
- une deuxième couche formée du revêtement 11 de la fibre optique ;
- une troisième couche formée de l'enrobage 130 en silicone ;

Le deuxième tronçon est destiné à pénétrer dans la zone intracérébrale. C'est sur ce deuxième tronçon que la fibre optique est dénudée pour retirer sa gaine de protection 12, permettant ainsi de rendre ce tronçon plus souple et étanche.

La sonde comporte un troisième tronçon (section S30) prolongeant le deuxième tronçon dans le sens allant de l'entrée optique vers la sortie optique de la sonde.

Ce troisième tronçon comporte uniquement :
- une première couche formée de l'enrobage 130 en silicone ;

Dans cette réalisation, ce troisième tronçon S30 comporte l'extrémité de la sonde formant la sortie optique.

Ce troisième tronçon S30 se termine avantageusement par une extrémité atraumatique. La lumière issue du coeur de la fibre optique traverse ce troisième tronçon. Selon l'application visée, ce troisième tronçon peut comporter ou faire office de diffuseur optique.

Afin de garantir le bon positionnement de la fibre au centre de la sonde, des espaceurs (anneaux, spirales...) peuvent être positionnés régulièrement le long de la fibre. Ces espaceurs seront préférentiellement réalisés dans le même matériau que le surmoulage en silicone 130.

Dans les deux réalisations, il faut comprendre que le premier tronçon (sections S1, S10) et le deuxième tronçon (sections S2, S20) permettent à la sonde 1 de disposer d'une architecture qui est à la fois suffisamment souple et flexible dans la zone implantée pour éviter tout impact ou traumatisme sur les tissus et suffisamment rigide à l'extérieur de la zone implantée pour éviter toute rupture.

Selon un aspect particulier de l'invention, l'enrobage 13, 130 et la gaine de protection 12 présentent des caractéristiques mécaniques distinctes et leurs fonctions sont également bien distinctes. La couche d'enrobage permet notamment d'apporter à la sonde des caractéristiques d'étanchéité que ne peut apporter la gaine de protection de la fibre optique et de conférer à la sonde son extrémité atraumatique.

De manière non limitative, pour les différents composants de la sonde, on aura les caractéristiques mécaniques suivantes :
- ETFE : Dureté de 62 à 72 Shore D, Module d'Young allant de 300 à 800MPa.
- Silicone : Dureté de 30 à 80 Shore A (préférentiellement 50), Module d'Young allant de 1 à 2MPa.

Par ailleurs, le retrait de la gaine de protection 12 sur une partie de la longueur de la sonde, comme proposé ci-dessus, peut présenter plusieurs avantages. Un premier avantage réside dans l'amélioration de la flexibilité de la sonde au niveau du tronçon dénudé. Un deuxième avantage réside dans l'amélioration de l'étanchéité, permettant d'éviter toute introduction de liquide. En effet, il s'avère notamment que la silicone est un matériau qui adhère mieux à la couche de revêtement de la fibre optique qu'à sa gaine de protection (en ETFE).

Pour faciliter la détermination de la quantité de lumière en sortie de la sonde, un bon positionnement en longueur et en centrage de la fibre dans la sonde est nécessaire. De manière avantageuse, le deuxième tronçon peut ainsi comporter un organe de centrage 17 du coeur 10 de la fibre et de son revêtement 11, positionné autour de la fibre optique dénudée. Cet organe de centrage peut être réalisé en matériau radio-opaque (par exemple : platine, inox) afin de permettre un contrôle précis du positionnement de la sonde par radiographie pendant la chirurgie ou faire un suivi postchirurgical.

Dans la première réalisation (section S2'), cet organe de centrage 17 est agencé pour prendre appui d'une part contre le revêtement 11 de la fibre optique et d'autre part pour être séparée du tube 14 par une couche d'enrobage 13 en silicone.

Dans la deuxième réalisation (section S20'), la silicone 130 vient enrober également l'organe de centrage 17, en plus de la fibre optique.

Cet organe de centrage permettra notamment de jouer le rôle de cale pour la fibre optique (10+11) que ce soit dans le tube 14 lors du processus de remplissage ou lors du surmoulage.

L'organe de centrage 17 peut comporter un manchon 170 présentant un canal central 171 longitudinal dans lequel sont insérés le coeur de la fibre optique et son revêtement (figures 3A et 3B).

Le manchon peut présenter une ou plusieurs rainures 172 latérales externes, par exemple longitudinale (figure 3B) ou hélicoïdale. Chaque rainure 172 est agencée pour permettre l'écoulement de la silicone dans le tube 14 lors du remplissage dans le cas de la première réalisation et pour servir d'ancrage dans le cas de la deuxième réalisation.

L'organe de centrage 17 peut comporter une pièce 173 en forme d'hélice (désignée cale spiralée ci-dessous) venant s'enrouler autour du coeur 10 de la fibre optique et de son revêtement 11 (figure 3C). Cette réalisation sera particulièrement avantageuse lorsque la sonde est mise en oeuvre par surmoulage et que la cale spiralée est réalisée en silicone. Pour faciliter la fabrication de la sonde en garantissant le centrage de la fibre et la faisabilité du surmoulage, sans faire de reprise sur la pièce finale, cette cale peut être préalablement réalisée et assemblée sur la fibre optique avant mise en place dans le moule de surmoulage. La cale spiralée permettra également de conserver une relative souplesse sur toute la longueur de la sonde.

De manière non limitative, la cale spiralée, en matériau bio-compatible, peut être réalisée en fil d'inox, platine irridié, ou par extrusion de silicone ou acrylate.

Cette cale spiralée peut présenter une section carrée, rectangulaire ou une section sensiblement carrée dont les deux côtés opposés sont recourbés. L'un de ces deux côtés présente ainsi un rayon de courbure adapté au diamètre externe de la fibre optique (coeur+revêtement) et l'autre de ces deux côtés peut présenter un rayon de courbure adapté au diamètre interne du tube 14. Cette solution permettra notamment de mieux épouser les contours du tube et de la fibre et limiter les risques d'amorces de rupture.

La cale spiralée peut être d'une longueur variable. Elle peut s'étendre du tronçon de connexion jusqu'à l'extrémité de la fibre optique, du côté distal. Côté distal, elle peut s'interrompre au même niveau que la fibre optique de stimulation ou en amont par rapport au sens optique.

L'organe de centrage 17 sera avantageusement réalisé avec un marquage radio-opaque de manière à permettre un contrôle du bon positionnement de la sonde lors de l'installation en chirurgie.

Le dispositif peut comporter au moins un système de retour optique permettant de transmettre une mesure de la puissance optique générée en bout de sonde. Cette solution de retour optique peut être employée dans le cadre de la première réalisation et de la deuxième réalisation.

Ce système comporte au moins une fibre optique 18 avantageusement enroulée en hélice autour de la fibre optique (10, 11) de stimulation, pour récupérer un signal optique de la sortie optique vers l'entrée optique. Elle assurera ainsi en complément ou en alternative le rôle de l'organe de centrage tel que défini ci-dessus et représenté sur la figure 3C et sa forme ne constituera pas une entrave à l'écoulement de la silicone 13 lors du surmoulage. Du côté de l'entrée optique, la fibre optique de retour est avantageusement prise dans la férule et est assemblée dans le connecteur optique qui est destiné à venir se connecter dans l'embase optique.

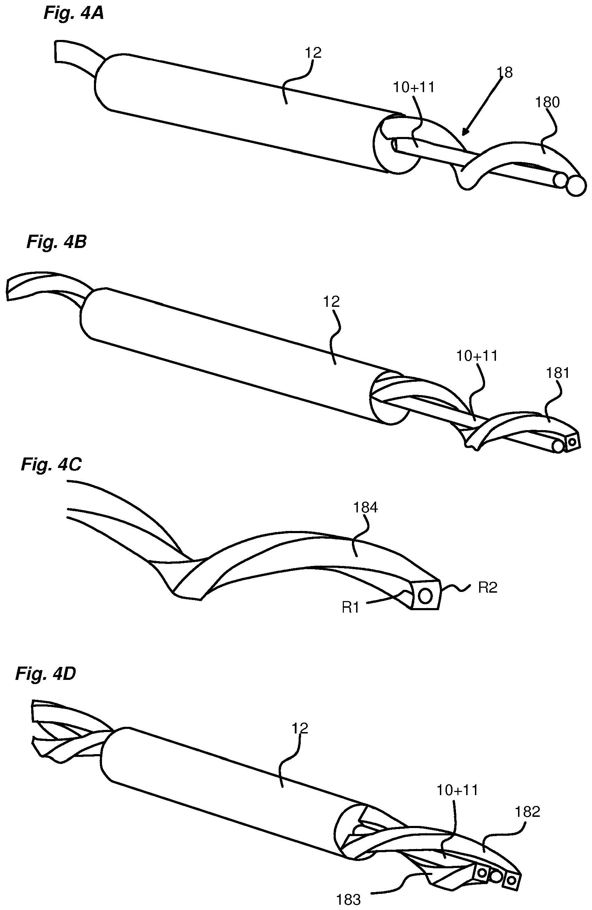

Cette fibre optique 18 de retour peut présenter différentes configurations :
- un coeur de section circulaire et un revêtement de section circulaire (180 - figure 4A) ;
- un coeur de section circulaire et un revêtement ayant une section de forme rectangle, avantageusement de forme carrée (181 - figure 4B) ;
- un coeur de section circulaire et un revêtement présentant une section sensiblement carrée, dont les deux côtés opposés sont recourbés (184 - figure 4C). L'un de ces deux côtés peut présenter ainsi un rayon de courbure R1 adapté au rayon de courbure externe de la fibre optique (coeur+revêtement) et l'autre de ces deux côtés peut présenter un rayon de courbure R2 adapté au rayon de courbure interne du tube 14. Cette solution permettra notamment de mieux épouser les contours du tube et de la fibre et de limiter les risques d'amorce de rupture.

Le système peut également comporter deux fibres optiques 182, 183 de retour, enroulées en hélice autour de la fibre optique de stimulation (figure 4D). Les deux fibres optiques pourront avoir une section de forme et/ou de dimensions identiques ou non, par exemple avec un coeur de section circulaire et un revêtement tel que défini ci-dessus en liaison avec les figures 4A à 4C. De même, elles pourront compléter un organe de centrage existant ou le remplacer pour maintenir et centrer la fibre optique lors de la fabrication.

Chaque fibre optique de retour comporte une extrémité optique d'entrée par laquelle le signal optique est capté et une extrémité optique de sortie par laquelle le signal optique capté est récupéré.

Les deux hélices formées par les deux fibres optiques présentent avantageusement un pas d'hélice identique et sont entrelacées autour de la fibre optique de stimulation.

Les deux fibres 182, 183 pourront se terminer à une distance différente de l'extrémité distale de la sonde afin d'évaluer le retour optique à différentes distances du point d'émission. Du côté proximal, les deux fibres optiques de retour sont avantageusement prises dans la férule et assemblées dans le connecteur optique qui est destiné à venir se connecter dans l'embase optique.

La fibre optique de stimulation et les deux fibres optiques de retour sont noyées au moins sur une partie ou sur la totalité de la longueur de la sonde dans l'enrobage de silicone, préférentiellement sur toute la longueur de la sonde, que ce soit dans le cadre de la première réalisation ou dans le cadre de la deuxième réalisation.

Selon un aspect particulier de l'invention, la solution à cale spiralée (sous la forme d'une fibre optique de retour ou non) permet notamment de limiter le glissement des deux éléments l'un contre l'autre, notamment lorsque la sonde est coudée.

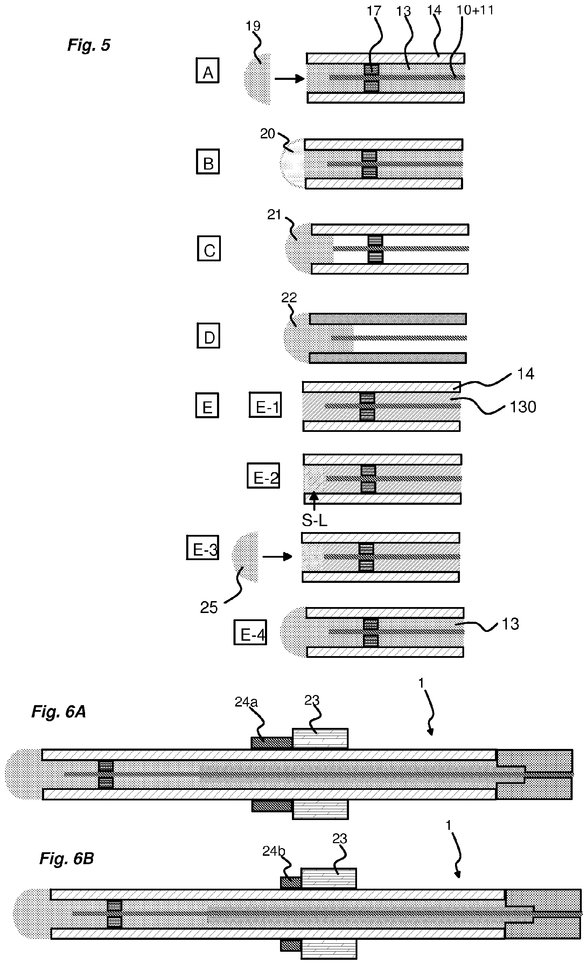

En référence à la figure 5, dans la première réalisation à tube 14, plusieurs solutions peuvent être envisagées pour former le dernier tronçon (section S4) du dispositif, à son extrémité distale :
- A : Une première solution consiste à venir coller une pièce 19 pleine en silicone de forme hémisphérique (ou oblongue) à l'extrémité du troisième tronçon (section S3) ; le centrage de la fibre peut être réalisé par un organe de centrage 17 spécifique tel que décrit ci-dessus ;
- B : Une deuxième solution consiste à déposer par tension de surface une goutte 20 de silicone sur l'extrémité du troisième tronçon ; Le centrage de la fibre peut être réalisé par un organe de centrage 17 spécifique ; Cette solution peut être mise en oeuvre lors du remplissage du tube ou a posteriori.
- C : Une troisième solution consiste à réaliser une pièce 21 pleine en silicone (époxy ou tout autre matériau stable dans les tissus) par moulage de manière à former un bouchon de qualité optique venant s'enfoncer dans le tube du deuxième tronçon jusqu'à l'extrémité de la fibre optique ; le centrage de la fibre (coeur+revêtement) peut être réalisé par un organe de centrage 17 spécifique ;
- D : Une quatrième solution consiste à réaliser une pièce 22 pleine en silicone (époxy ou tout autre matériau stable dans les tissus - transparente ou diffusante selon l'application visée) par moulage et à la rapporter par collage de manière à s'enfoncer dans le tube jusqu'à venir entourer l'extrémité de la fibre ; la pièce comporte également un canal interne dans lequel peut s'enfoncer l'extrémité de la fibre (coeur + revêtement), jouant alors le rôle de l'organe de centrage ; Le tube 14 pourra être de type radio-opaque.
- E : une cinquième solution consiste à réaliser un dôme silicone cohésif au remplissage silicone du tube ; ainsi, la partie distale de la sonde serait réalisée en même temps que le remplissage du tube. Pour rendre cela réalisable, il convient de remplir le tube (E-1) sur toute sa longueur avec la silicone non réticulée 130 (le tube loge déjà la fibre optique de stimulation, l'organe de centrage et les éventuelles fibres optiques de retour), de figer le remplissage silicone du tube par séchage localisé (S-L) avec un pistolet à air chaud par exemple) au-delà de la bague de centrage (E-2) du côté remplissage, pour éviter que la silicone s'écoule du tube à l'étape de réalisation du dôme, puis de découper l'extrémité du tube à la longueur souhaitée (étape optionnelle), et de déposer une quantité de silicone calibrée 25 afin de former la partie hémisphérique (E-3). Dans un deuxième temps, c'est l'intégralité de la sonde et du dôme qui est réticulée (par exemple en étuve), rendant ainsi le dôme cohésif de l'intérieur de la sonde (E-4). Cette solution présente l'avantage de garantir une très bonne cohésion de la sonde. On minimise le risque existant de détachement du dôme dans le cas d'un dôme rapporté de façon adhésive, qui aurait représenté un danger notamment en cas d'implantation intracérébrale.

Dans la première réalisation de la sonde optique, selon une particularité, la distance (L) entre l'extrémité du tube 13 et l'extrémité du coeur de la fibre optique du côté distal permet de jouer sur la densité de puissance à l'extrémité du dispositif. Le tube étant réalisé en silicone non-optique, il faudra veiller à ce que le troisième tronçon (section S3) ne soit pas trop long afin d'éviter que le rayon lumineux sortant de la fibre optique (au niveau du deuxième tronçon - section S2) ne vienne frapper la paroi interne du tube 14. Cette distance sera par exemple de 2,5mm (avec une variation possible de +/-0,25mm).

Le dispositif peut comporter un système de butée destiné à régler le niveau d'enfoncement de la sonde lors de l'implantation (figures 6A et 6B). Cette solution permet une mise en place manuelle de la sonde, sans risque pour le patient, et s'applique aux deux réalisations du dispositif.

Ce système de butée est destiné à venir en appui contre la surface du Neuroguide préalablement positionné et fixé sur la boite crânienne lors de l'implantation et permet ainsi de stopper et de régler le niveau d'enfoncement du dispositif.

Ce système peut être collé ou intégré à la sonde optique selon le mode de réalisation.

Ce système comporte un organe, tel qu'un manchon 23, fixé sur le tube 14 du dispositif ou sur l'enrobage 130 de surmoulage et une ou plusieurs entretoises 24a, 24b de longueurs différentes disposés entre ledit organe et la zone extra-corticale pour ajuster la longueur d'enfoncement de la sonde. La figure 6A représente ainsi une première configuration avec une entretoise 24a d'une première longueur et la figure 6B représente une deuxième configuration avec une entretoise 24b d'une longueur distincte de celle de la première configuration. En jouant sur la longueur de l'entretoise, il est ainsi possible de régler la position de la butée formée par le manchon 23 contre une autre butée.

Le manchon 23 pourra être réalisé dans différentes matières, silicone, métal (titane, inox), ou plastique (PEEK pour PolyEtherEtherKetone)...

L'invention présente ainsi de nombreux avantages, parmi lesquels :
- Une sonde parfaitement adaptée à une implantation long terme, à la fois étanche, solide et souple dans la zone particulièrement sensible ;
- L'utilisation de différents types de matériaux de qualité optique (silicone, PU...) tels que décrits ci-dessus, permet la réalisation d'un dispositif compatible avec une mise en place dans le cerveau et particulièrement dans le troisième ventricule en passant par un des ventricules latéraux ;
- Une solution facile à fabriquer, que cela soit par injection dans le tube ou par surmoulage ;
- Une solution fiable, obtenue notamment grâce à un parfait positionnement des différents éléments, grâce à l'organe de centrage ;
- Une sonde permettant un traitement efficace, notamment grâce à la solution de retour optique, cette dernière étant par ailleurs particulièrement compacte ;

## Revendications

1. Dispositif d'illumination implantable, destiné à être implanté dans un être vivant pour illuminer de manière localisée une zone dudit être vivant, ledit dispositif comportant une sonde (1) présentant une architecture allongée suivant un axe entre une extrémité proximale et une extrémité distale, ladite sonde se présentant sous la forme d'une tige de section circulaire de diamètre constant sur toute sa longueur, ladite sonde (1) comportant :
- une fibre optique de stimulation qui comporte un coeur (10), un revêtement (11) et une gaine de protection (12),
- un enrobage (13, 130), et un tube (14),
- et ladite architecture comportant :
- plusieurs tronçons successifs suivant son axe longitudinal, chaque tronçon présentant une section (Sc à S4 ou Sc à S30) à plusieurs couches superposées de manière concentrique, dans lesquelles chaque tronçon comporte une section à plusieurs couches superposées distincte de la section de chaque tronçon adjacent,
- au moins un premier tronçon le long duquel ladite fibre optique de stimulation est enrobée par l'enrobage (13, 130),
- au moins un deuxième tronçon prolongeant ledit premier tronçon, dans lequel ladite fibre optique de stimulation est dénudée pour retirer sa gaine de protection de manière à laisser uniquement son coeur et son revêtement optique enrobé par l'enrobage,
- au moins un tronçon terminal dont la section (S4, S30) comporte uniquement ledit enrobage (13), ledit tronçon terminal comportant l'extrémité distale de la sonde et en ce que cette extrémité distale est configurée de manière à être atraumatique,
- la section (S1) du premier tronçon comportant une première couche comprenant le coeur (10) de fibre optique, une deuxième couche comprenant le revêtement (11) de fibre optique, une troisième couche comprenant la gaine de protection (12) de fibre optique, une quatrième couche comprenant l'enrobage (13) et une cinquième couche externe composée du tube (14),
- la section (S2) du deuxième tronçon comportant une première couche comprenant le coeur (10) de ladite fibre optique, une deuxième couche formée du revêtement (11) de la fibre optique, une troisième couche comprenant ledit enrobage (13) et une quatrième couche comprenant ledit tube (14),
- le dispositif comportant un troisième tronçon prolongeant ledit deuxième tronçon, dont la section (S3) comporte une première couche comprenant ledit enrobage (13) et une deuxième couche comprenant ledit tube (14).

2. Dispositif selon la revendication 1, **caractérisé en ce qu'**il comporte un tronçon de connexion, situé en amont du premier tronçon, dont la section (Sc) comporte une première couche formée du coeur de la fibre, une deuxième couche formée du revêtement de fibre optique, une troisième couche formée d'une colle et une quatrième couche formée d'une férule.

3. Dispositif selon l'une des revendications 1 à 2, **caractérisé en ce qu'**il comporte au moins un organe de centrage (17) de ladite fibre optique de stimulation dénudée, positionné autour de ladite fibre optique et agencé pour former une cale pour ladite fibre optique dénudée.

4. Dispositif selon la revendication 3, **caractérisé en ce que** ledit organe de centrage comporte un manchon cylindrique présentant un canal central dans lequel est insérée ladite fibre optique dénudée et **en ce que** ledit manchon comporte une ou plusieurs rainures (172) latérales externes.

5. Dispositif selon la revendication 3, **caractérisé en ce que** ledit organe de centrage comporte une pièce (173) en forme d'hélice venant s'enrouler autour du coeur (10) de la fibre optique et de son revêtement (11).

6. Dispositif selon l'une des revendications 1 à 5, **caractérisé en ce qu'**il comporte un système de retour optique comprenant au moins une première fibre optique de retour comprenant une extrémité optique d'entrée configurée pour capter un signal optique et une extrémité optique de sortie configurée pour transmettre le signal optique capté,
- ladite première fibre optique de retour étant enroulée en hélice autour de la fibre optique de stimulation sur au moins une partie de la longueur de ladite fibre optique de stimulation.

7. Dispositif selon la revendication 6, **caractérisé en ce que** le système de retour optique comporte une deuxième fibre optique de retour enroulée en hélice autour de ladite fibre optique de stimulation et comportant une extrémité optique d'entrée et une extrémité optique de sortie.

8. Dispositif selon la revendication 6 ou 7, **caractérisé en ce que** l'extrémité optique d'entrée de la première fibre optique de retour et l'extrémité optique d'entrée de la deuxième fibre optique de retour sont situées à des longueurs distinctes de la sortie optique de la fibre optique de stimulation.

9. Dispositif selon l'une des revendications 6 à 8, **caractérisé en ce que** chaque fibre optique de retour comporte un coeur de section circulaire et un revêtement optique présentant une section sensiblement carrée, présentant deux côtés opposés recourbés, l'un de ces deux côtés présentant un premier rayon de courbure (R1) et l'autre de ces deux côtés opposés présentant un deuxième rayon de courbure (R2) distinct du premier rayon de courbure.

10. Procédé de fabrication d'un dispositif d'illumination implantable tel que défini dans la revendication 1, **caractérisé en ce qu'**il comporte les étapes suivantes :
- Remplissage du tube (14) par le matériau d'enrobage non réticulé (130),
- Séchage au moins partiel du matériau d'enrobage non réticulé (130) pour le figer à l'intérieur du tube (14),
- Dépôt d'une quantité calibrée (25) de matériau d'enrobage non réticulé pour former ledit tronçon terminal,
- Réticulation du matériau d'enrobage sur toute la longueur de la sonde.

## Patentansprüche

1. Implantierbare Beleuchtungsvorrichtung, die dazu bestimmt ist, in ein Lebewesen implantiert zu werden, um einen Bereich des Lebewesens lokal zu beleuchten, wobei die Vorrichtung eine Sonde (1) umfasst, die eine längliche Architektur entlang einer Achse zwischen einem proximalen Ende und einem distalen Ende aufweist, wobei die Sonde in der Form einer Stange mit kreisförmigem Querschnitt mit über die gesamte Länge konstantem Durchmesser vorliegt, wobei die Sonde (1) umfasst:
- einen Lichtwellenleiter zur Stimulation, welcher einen Kern (10), eine Beschichtung (11) und einen Schutzmantel (12) umfasst,
- eine Umhüllung (13, 130) und ein Rohr (14),
- und wobei die Architektur umfasst:
- mehrere entlang ihrer Längsachse aufeinander folgende Abschnitte, wobei jeder Abschnitt einen Querschnitt (Sc bis S4 oder Sc bis S30) mit mehreren konzentrisch übereinanderliegenden Schichten aufweist, wobei jeder Abschnitt einen Querschnitt mit mehreren übereinanderliegenden Schichten aufweist, der vom Querschnitt jedes benachbarten Abschnitts verschieden ist,
- mindestens einen ersten Abschnitt, entlang dem der Lichtwellenleiter zur Stimulation mit der Umhüllung (13, 130) umhüllt ist,
- mindestens einen zweiten Abschnitt, der den ersten Abschnitt verlängert und auf dem der Lichtwellenleiter zur Stimulation abisoliert wurde, indem sein Schutzmantel abgezogen wurde, so dass nur sein Kern und seine von der Umhüllung umhüllte optische Beschichtung verbleiben,
- mindestens einen Endabschnitt, dessen Querschnitt (S4, S30) nur die Umhüllung (13) umfasst, wobei der Endabschnitt das distale Ende der Sonde umfasst, und dadurch, dass dieses distale Ende so ausgebildet ist, dass es atraumatisch ist,
- wobei der Querschnitt (S1) des ersten Abschnitts eine erste Schicht, die den Kern (10) des Lichtwellenleiters umfasst, eine zweite Schicht, die die Beschichtung (11) des Lichtwellenleiters umfasst, eine dritte Schicht, die den Schutzmantel (12) des Lichtwellenleiters umfasst, eine vierte Schicht, die die Umhüllung (13) umfasst, und eine äußere fünfte Schicht, die aus dem Rohr (14) besteht, aufweist,
- wobei der Querschnitt (S2) des zweiten Abschnitts eine erste Schicht, die den Kern (10) des Lichtwellenleiters umfasst, eine zweite Schicht, die von der Beschichtung (11) des Lichtwellenleiters gebildet wird, eine dritte Schicht, die die Umhüllung (13) umfasst, und eine vierte Schicht, die das Rohr (14) umfasst, aufweist,
- wobei die Vorrichtung einen dritten Abschnitt, der den zweiten Abschnitt verlängert, aufweist, dessen Querschnitt (S3) eine erste Schicht, die die Umhüllung (13) umfasst, und eine zweite Schicht, die das Rohr (14) umfasst, aufweist.

2. Vorrichtung nach Anspruch 1, **dadurch gekennzeichnet, dass** sie einen Anschlussabschnitt umfasst, der sich vor dem ersten Abschnitt befindet und dessen Querschnitt (Sc) eine erste Schicht, die vom Kern des Leiters gebildet wird, eine zweite Schicht, die von der Beschichtung des Lichtwellenleiters gebildet wird, eine dritte Schicht, die von einem Klebstoff gebildet wird, und eine vierte Schicht, die von einer Quetschhülse gebildet wird, umfasst.

3. Vorrichtung nach einem der Ansprüche 1 bis 2, **dadurch gekennzeichnet, dass** sie mindestens ein Organ zur Zentrierung (17) des abisolierten Lichtwellenleiters zur Stimulation umfasst, das um den Lichtwellenleiter herum positioniert ist und dazu eingerichtet ist, ein Distanzstück für den abisolierten Lichtwellenleiter zu bilden.

4. Vorrichtung nach Anspruch 3, **dadurch gekennzeichnet, dass** das Zentrierorgan eine zylindrische Hülse umfasst, die einen zentralen Kanal aufweist, in welchen der abisolierte Lichtwellenleiter eingesteckt wird, und dadurch, dass die Hülse eine oder mehrere äußere seitliche Rillen (172) umfasst.

5. Vorrichtung nach Anspruch 3, **dadurch gekennzeichnet, dass** das Zentrierorgan ein schraubenlinienförmiges Teil (173) umfasst, das sich um den Kern (10) des Lichtwellenleiters und um dessen Beschichtung (11) wickelt.

6. Vorrichtung nach einem der Ansprüche 1 bis 5, **dadurch gekennzeichnet, dass** sie ein optisches Rückführungssystem aufweist, das mindestens einen ersten zurückführenden Lichtwellenleiter umfasst, der eine Lichteintrittsende, das dafür ausgelegt ist, ein optisches Signal zu erfassen, und ein Lichtaustrittsende, das dafür ausgelegt ist, das erfasste optische Signal zu übertragen, umfasst,
- wobei der erste zurückführende Lichtwellenleiter auf wenigstens einem Teil der Länge des Lichtwellenleiters zur Stimulation schraubenlinienförmig um den Lichtwellenleiter zur Stimulation gewickelt ist.

7. Vorrichtung nach Anspruch 6, **dadurch gekennzeichnet, dass** das optische Rückführungssystem einen zweiten zurückführenden Lichtwellenleiter aufweist, der schraubenlinienförmig um den Lichtwellenleiter zur Stimulation gewickelt ist und ein Lichteintrittsende und ein Lichtaustrittsende aufweist.

8. Vorrichtung nach Anspruch 6 oder 7, **dadurch gekennzeichnet, dass** sich das Lichteintrittsende des ersten zurückführenden Lichtwellenleiters und das Lichteintrittsende des zweiten zurückführenden Lichtwellenleiters in unterschiedlichen Abständen vom optischen Ausgang des Lichtwellenleiters zur Stimulation befinden.

9. Vorrichtung nach einem der Ansprüche 6 bis 8, **dadurch gekennzeichnet, dass** jeder zurückführende Lichtwellenleiter einen Kern mit kreisförmigem Querschnitt und eine optische Beschichtung, die einen im Wesentlichen quadratischen Querschnitt aufweist, der zwei gekrümmte gegenüberliegende Seiten aufweist, umfasst, wobei die eine dieser zwei Seiten einen ersten Krümmungsradius (R1) und die andere dieser zwei gegenüberliegenden Seiten einen zweiten Krümmungsradius (R2), der vom ersten Krümmungsradius verschieden ist, aufweist.

10. Verfahren zur Herstellung einer implantierbaren Beleuchtungsvorrichtung, wie in Anspruch 1 definiert, **dadurch gekennzeichnet, dass** es die folgenden Schritte umfasst:
- Füllen des Rohres (14) mit dem nicht vernetzten Umhüllungsmaterial (130),
- wenigstens teilweises Trocknen des nicht vernetzten Umhüllungsmaterials (130), um es im Inneren des Rohres (14) erstarren zu lassen,
- Abscheidung einer kalibrierten Menge (25) von nicht vernetztem Umhüllungsmaterial, um den Endabschnitt auszubilden,
- Vernetzung des Umhüllungsmaterials auf der gesamten Länge der Sonde.

## Claims

1. Implantable illuminating device intended to be implanted in a living being with a view to locally illuminating a region of said living being, said device comprising a probe (1) having an architecture that is elongate along an axis between a near end and a far end, said probe taking the form of a rod of circular section of constant diameter over all its length, said probe (1) comprising:
- a stimulating optical fibre that comprises a core (10), a cladding (11) and a protective coating (12),
- a jacket (13, 130), and a tube (14),
- said architecture comprising:
- a plurality of successive segments along its longitudinal axis, each segment having a section (Sc to S4 or Sc to S30) containing a plurality of concentrically superposed layers, in which each segment comprises a section containing a plurality of superposed layers that is different from the section of each adjacent segment,
- at least a first segment along which said stimulating optical fibre is jacketed by the jacket (13, 130),
- at least a second segment extending said first segment, in which said stimulating optical fibre is stripped to remove its protective coating so as to leave behind only its core and its optical cladding jacketed by the jacket,
- at least a terminal segment the section (S4, S30) of which comprises only said jacket (13), said terminal segment comprising the far end of the probe and in that this far end is configured so as to be atraumatic,
- the section (S1) of the first segment comprising a first layer comprising the optical-fibre core (10), a second layer comprising the optical-fibre cladding (11), a third layer comprising the protective optical-fibre coating (12), a fourth layer comprising the jacket (13) and an external fifth layer composed of the tube (14),
- the section (S2) of the second segment comprising a first layer comprising the core (10) of said optical fibre, a second layer formed from the cladding (11) of the optical fibre, a third layer comprising said jacket (13) and a fourth layer comprising said tube (14),
- the device comprising a third segment extending said second segment and the section (S3) of which comprises a first layer comprising said jacket (13) and a second layer comprising said tube (14).

2. Device according to Claim 1, **characterized in that** it comprises a connecting segment, located upstream of the first segment, the section (Sc) of which comprises a first layer formed from the core of the fibre, a second layer formed from the optical-fibre cladding, a third layer formed from an adhesive and a fourth layer formed from a ferrule.

3. Device according to one of Claims 1 to 2, **characterized in that** it comprises at least one member (17) for centring said stripped stimulating optical fibre, said member being positioned around said optical fibre and arranged to form a shim for said stripped optical fibre

4. Device according to Claim 3, **characterized in that** said centring member comprises a cylindrical sleeve having a central channel in which is inserted said stripped optical fibre, and **in that** said sleeve comprises one or more external lateral grooves (172).

5. Device according to Claim 3, **characterized in that** said centring member comprises a part (173) of helical shape that is wound around the core (10) of the optical fibre and its cladding (11).

6. Device according to one of Claims 1 to 5, **characterized in that** it comprises an optical feedback system comprising at least a first feedback optical fibre comprising an input optical end configured to capture an optical signal and an output optical end configured to transmit the captured optical signal,
- said first feedback optical fibre being wound helically around the stimulating optical fibre over at least one portion of the length of said stimulating optical fibre.

7. Device according to Claim 6, **characterized in that** the optical feedback system comprises a second feedback optical fibre wound helically around said stimulating optical fibre and comprising an input optical end and an output optical end.

8. Device according to Claim 6 or 7, **characterized in that** the input optical end of the first feedback optical fibre and the input optical end of the second feedback optical fibre are located at different lengths from the optical output of the stimulating optical fibre.

9. Device according to one of Claims 6 to 8, **characterized in that** each feedback optical fibre comprises a core of circular section and an optical cladding having a substantially square section, having two curved opposite sides, one of these two sides having a first radius of curvature (R1) and the other of these two opposite sides having a second radius of curvature (R2) different from the first radius of curvature.

10. Process for fabricating an implantable illuminating device such as defined in Claim 1, **characterized in that** it comprises the following steps:
- filling the tube (14) with the un-crosslinked jacket material (130),
- at least partially drying the un-crosslinked jacket material (130) in order to set it inside the tube (14),
- depositing a calibrated amount (25) of un-crosslinked jacket material in order to form said terminal segment,
- crosslinking the jacket material over the entire length of the probe.
